(19) Europäisches Patentamt
European Patent Office
Office européen des brevets

(11) Veröffentlichungsnummer: **0 179 740**
**B1**

(12) # EUROPÄISCHE PATENTSCHRIFT

(45) Veröffentlichungstag der Patentschrift: **20.04.88**

(51) Int. Cl.[4]: **C 07 C 121/78,** C 07 C 120/00

(21) Anmeldenummer: **85810482.1**

(22) Anmeldetag: **21.10.85**

(54) **Verfahren zur Herstellung von 1,4-Diamino-2,3-dicyanoanthrachinon.**

(30) Priorität: **26.10.84 CH 5121/84**

(43) Veröffentlichungstag der Anmeldung:
**30.04.86 Patentblatt 86/18**

(45) Bekanntmachung des Hinweises auf die Patenterteilung:
**20.04.88 Patentblatt 88/16**

(84) Benannte Vertragsstaaten:
**CH DE FR GB LI**

(56) Entgegenhaltungen:
**EP - B - 0 023 645**
**DE - A - 3 039 262**
**DE - B - 1 108 704**

(73) Patentinhaber: **CIBA-GEIGY AG, Klybeckstrasse 141, CH-4002 Basel (CH)**

(72) Erfinder: **Schaulin, Rudolf, Dr., Schäferstrasse 62, CH-4125 Riehen (CH)**
Erfinder: **Grélat, Maurice, Dr., Via al Doyro 3, CH-6815 Melide (CH)**

EP 0 179 740 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von 1,4-Diamino-2,3-dicyanoanthrachinon ausgehend von 1,4-Diaminoanthrachinon oder 1,4-Diaminoanthrachinonmono- oder -disulfonsäure oder deren Salzen.

1,4-Diamino-2,3-dicyanoanthrachinon ist in wichtiges Zwischenprodukt für die Herstellung von Textilfarbstoffen. In der Vergangenheit sind eine ganze Reihe von Verfahren zur Herstellung des Diamino-dicyanoanthrachinons entwickelt worden. Ausgangsmaterial ist allgemein das 1,4-Diaminoanthrachinon, die 1,4-Diaminoanthrachinon-2-sulfonsäure oder auch 1,4-Diamino-2,3-dihalogeno-anthrachinone, die mit Cyaniden zum Diamino-dicyanoanthrachinon umgesetzt werden. Im wesentlichen unterscheiden sich diese bekannten Verfahren hinsichtlich des Lösungsmittels, in dem die Reaktion durchgeführt wird und gegebenenfalls spezifischer Zusätze. Solche spezifische Zusätze sind z.B. quaternäre Ammoniumverbindungen (DE-A-3 039 262), die bei der Umsetzung der Ausgangsmaterialien mit einer Cyanidionen abgebenden Verbindung eingesetzt werden, um die Bildung von Nebenprodukten gering zu halten. Was die Lösungsmittel anbetrifft, so wird entweder in Wasser gearbeitet (z.B. DE-B-1 108 704) oder wasserfrei in einem aprotischen organischen Lösungsmittel, wie z.B. Dimethylsulfoxid oder Dimethylformamid (DE-A-2 524 748) oder in Formamid bzw. N-Methylformamid (EP-A-23 645).

Beide Verfahrensvarianten sind mit Nachteilen behaftet. So ist die Reaktion bei Verwendung von Wasser als Reaktionsmedium nur in hoher Verdünnung durchführbar, was eine niedrige Raum-Zeit-Ausbeute zur Folge hat. In wasserfreien organischen Lösungsmitteln kann hingegen sehr konzentriert gearbeitet werden, derartige Verfahren sind jedoch relativ aufwendig, da nicht nur das Lösungsmittel sondern auch die Ausgangsmaterialien vor der Reaktion getrocknet werden müssen. Hinzu kommt, dass man das organische Lösungsmittel am Ende der Reaktion wieder regenerieren muss.

Gefunden wurde nun, dass die Cyanierung des Diaminoanthrachinons bzw. der Diaminoanthrachinonsulfonsäure bei Verwendung eines Carbonsäureamids als Lösungsmittel auch in Gegenwart von Wasser und in Abwesenheit quaternärer Ammoniumsalze durchgeführt werden kann. Es können sogar Gemische aus Carbonsäureamid und Wasser verwendet werden, die bis zu 90 Gewichtsprozent aus Wasser bestehen, ohne dass man dadurch wesentlich mehr Lösungsmittel benötigt, als beim Arbeiten in rein organischem Medium.

Gegenstand der Erfindung ist somit ein Verfahren zur Herstellung von 1,4-Diamino-2,3-dicyanoanthrachinon durch Umsetzen von Diaminoanthrachinonen der Formel

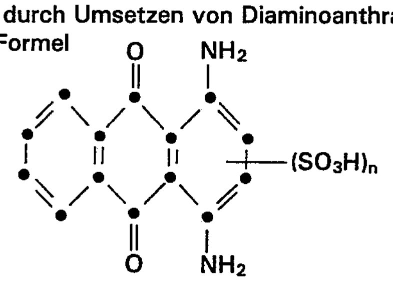

oder deren Salze, worin n 0, 1 oder 2 ist, mit einer Cyanidionen abgebenden Verbindung, in Gegenwart eines Oxidationsmittels, das dadurch gekennzeichnet ist, dass man als Lösungsmittel ein Gemisch aus einem Carbonsäureamid, einem N-Alkylcarbonsäureamid oder einem Lactam und Wasser verwendet, dessen Wassergehalt über 10 Gewichtsprozent liegt, und in Abwesenheit von quaternären Ammoniumsalzen arbeitet.

Dieses Verfahren hat den wesentlichen Vorteil, dass das als Ausgangsmaterial verwendete Diaminoanthrachinon als wasserfeuchter Presskuchen eingesetzt werden kann und nicht zuvor getrocknet werden muss. Zudem werden Carbonsäureamide, wie z.B. das Acetamid relativ leicht von Mikroorganismen abgebaut, so dass bei Verwendung von Lösungsmittelgemischen mit einem Wasseranteil von ca. 50% und mehr, diese am Ende der Reaktion nach der Aufarbeitung direkt in das Abwasser geleitet werden können und somit eine aufwendige Regenerierung des Lösungsmittels überflüssig ist. Darüberhinaus hat sich gezeigt, dass die Reaktion in einem Gemisch aus Carbonsäureamid bzw. Lactam und Wasser überraschenderweise schneller abläuft, als in einem wasserfreien Carbonsäureamid als alleinigem Lösungsmittel, wodurch im vorliegenden Fall eine höhere Raum-Zeit-Ausbeute erzielt wird. Hinzu kommt, dass die als Ausgangsmaterial bevorzugt eingesetzte Diaminoanthrachinonsulfonsäure und das Cyanid in einem wässrig-organischen Medium wesentlich leichter löslich sind als im rein organischen Medium.

Die als Ausgangsmaterial verwendeten Diaminoanthrachinone der angegebenen Formel sind bekannt. So wird beispielsweise die 1,4-Diaminoanthrachinon-2-sulfonsäure durch Umsetzen der 1-Amino-4-bromanthrachinon-2-sulfonsäure (Bromaminsäure) mit Ammoniak unter Druck erhalten (DE-PS 1 142 174 und DE-PS 1 155 786).

Wird das Verfahren mit Diaminoanthrachinonmono- oder -di-sulfonsäure als Ausgangsmaterial durchgeführt, so können sowohl die freie Säure, wie auch deren Salze, insbesondere die Alkali- und Ammoniumsalze eingesetzt werden. Als Ausgangsmaterial bevorzugt ist die 1,4-Diaminoanthrachinon-2--sulfonsäure.

Bei den im vorliegenden Verfahren als Lösunsmittel verwendeten Carbonsäureamiden handelt es sich in erster Linie um Amide von Monocarbonsäuren einer C-Kettenlänge von 1 bis 6. Genannt sind beispielsweise Formamid, Acetamid oder Propionamid. In Frage kommen ferner N-alkylierte Carbonsäureamide und zwar die N-monoalkylierten Amide, während sich die N,N-Dialkylcarbonsäureamide als ungeeignet erwiesen haben. Genannt sind hier z.B. das N-Methylformamid und das N-methylacetamid. Sehr gute Ergebnisse erzielt man indess, wenn als Lösungsmittel Lactam/Wasser-Gemische verwendet werden. Als Lactame kommen vor allem solche einer Ringgrösse von 5 bis 7 in Betracht; genannt sind 2-Pyrrolidon, 2-Piperidon sowie ε-Caprolactam. Als Lösungsmittel bevorzugt ist vor allem das Acetamid und insbesondere das 2-Pyrrolidon.

Durchgeführt wird das erfindungsgemässe Verfahren in einem Gemisch aus Carbonsäureamid bzw.

Lactam und Wasser. Die Menge an Wasser bezogen auf das Gemisch ist > 10 Gewichtsprozent und kann bis zu 90 Gewichtsprozent betragen, vorteilhaft verwendet man ein Carbonsäureamid bzw. Lactam/Wasser-Gemisch, das 10 bis 60 Gewichtsprozent Wasser enthält.

Die Menge an eingesetztem wasserhaltigem Lösungsmittel hängt im wesentlichen von der Löslichkeit des Ausgangsmaterials ab. Zweckmässigerweise wird soviel Lösungsmittel verwendet, dass das Ausgangsmaterial vollständig gelöst ist. Im allgemeinen benötigt man für 1 Teil Diaminoanthrachinon bzw. Diaminoanthrachinonsulfonsäure 4 bis 10 Teile wasserhaltiges Carbonsäureamid bzw. Lactam/Wasser-Gemisch.

Als Cyanidionen abgebende Verbindungen werden vor allem Alkalimetall- und Erdalkalimetallcyanide verwendet, wie z.B. Natrium-, Kalium- oder Magnesiumcyanid. In Frage kommen ferner Ammoniumcyanid, Blausäure, sowie Cynahydrine von Aldehyden und Ketonen. Bevorzugt sind Natrium- und Kaliumcyanid. Verwendet wird das Cyanidionen abgebende Mittel in einer Menge von vorteilhaft 2 bis 10 Mol pro Mol Ausgangsverbindung.

Das erfindungsgemässe Verfahren wird in Gegenwart eines Oxidationsmittels durchgeführt, dabei kommen sowohl anorganische wie organische Oxidationsmittel in Betracht. So z.B. Sauerstoff, Nitrile, Nitrate, Bromate, Persulfate, Wasserstoffperoxid, organische Persäuren, wie Peressigsäure, ferner Nitroaromaten, wie Nitrobenzol, m-Nitrobenzolsulfonsäure und deren Salze oder auch Nitrobenzoesäure. Von diesen Oxidationsmitteln bevorzugt ist das Nitrobenzol und die m-Nitrobenzolsulfonsäure.

Die Reaktionstemperatur liegt je nach Ausgangsverbindung zwischen 40 und 150°C, vorteilhaft zwischen 60 und 100°C.

Es hat sich als zweckmässig erwiesen, dem Reaktionsgemisch ein säurebindendes Mittel zuzusetzen, z.B. ein Alkali- oder Erdalkalimetallsalz einer schwachen Säure, etwa Natriumcarbonat, Kaliumcarbonat, Natriumhydrogencarbonat, Natriumacetat, Calciumcarbonat oder auch wässrige Ammoniaklösung oder Ammoniumhydrogencarbonat. Neben anorganischen Verbindungen sind auch organische Basen geeignet, wie beispielsweise Mono-, Di-, Trialkylamine mit einem gegebenenfalls durch Sauerstoffatome unterbrochenen $C_2$ bis $C_8$ Alkylrest, wie z.B. Butylamin, Triäthylamin oder Tris-(dioxa-3,6-heptyl)-amin, oder auch solche Alkylamine mit beispielsweise durch Hydroxy- oder Alkoxygruppen substituierten Alkylresten. Bevorzugt verwendet werden Soda, Natriumhydrogencarbonat, wässriges Ammoniak und/oder Mono-, Di- oder Trialkylamine. Arbeitet man auschliesslich unter Zusatz von Ammoniak als Base, so wird die Reaktion vorteilhaft unter Druck durchgeführt.

Die Reaktion lässt sich anhand des Dünnschichtchromatogramms leicht verfolgen und ist bereits nach 2 bis 5 Stunden beendet. Die Aufarbeitung gestaltet sich relativ einfach, da das Diamino-dicyano-anthrachinon im erfindungsgemäss verwendeten Carbonsäureamid bzw. Lactam/Wasser-Gemisch schwerer löslich ist, als die Ausgangsverbindung und daher leicht, z.B. durch Filtration oder Zentrifugieren abgetrennt werden kann. Man erhält nach vorliegendem Verfahren in guter Ausbeute ein Diaminodicyanoanthrachinon hoher Reinheit, das sich direkt zu anthrachinoiden Dispersfarbstoffen weiterverarbeiten lässt.

Das erfindungsgemässe Verfahren wird üblicherweise so durchgeführt, dass man das 1,4-Diaminoanthrachinon oder die 1,4-Diaminoanthrachinonsulfonsäure in wasserhaltigem Carbonsäureamid, beispielsweise Acetamid oder einem Lactam/Wassergemisch, z.B. Pyrrolidon/Wassergemisch mit einem Wassergehalt von 20 bis 50 Gewichtsprozent bei Raumtemperatur mit einem säurebindenden Mittel, wie z.B. Soda oder Natriumhydrogencarbonat, und/oder einem tertiären Amin vorlegt und anschliessend das Oxidationsmittel, z.B. m-Nitrobenzolsulfonsäure und das Cyanid, z.B. Natriumcyanid, zugibt. Vor Zusatz des säurebindenden Mittels wird der pH-Wert der Lösung zweckmässigerweise mit wässrigem Ammoniak neutral bis schwach eingestellt. Nach einer Reaktionszeit von ca. 30 Minuten bis 4 Stunden bei einer Temperatur von ca. 80°C ist die Reaktion beendet und das ausgefallene Produkt wird abfiltriert und gegebenenfalls mit etwas Wasser und/oder Methanol bzw. Äthanol gewaschen und anschliessend getrocknet. Das gewünschte Produkt wird nach diesem Verfahren mit einer Ausbeute von über 90% erhalten.

Wird als Ausgangsverbindung die 1,4-Diamino-anthrachinon-2-sulfonsäure verwendet, so kann diese in einem vorgelagerten Reaktionsschritt aus Bromaminsäure, wie eingangs erwähnt, durch Behandeln mit Ammoniak in Gegenwart eines Kupfersalzes im gleichen Lösungsmittel hergestellt und ohne Zwischenisolieren in einer Eintopfreaktion zum Diamino-dicyanoanthrachinon umgesetzt werden.

Die folgenden Beispiele dienen der Veranschaulichung der Erfindung; Teile bedeuten Gewichtsteile und Prozente Gewichtsprozente. Zur dünnschichtchromatographischen Analyse des Produktes werden DC-Fertigplatten, Kieselgel 60 F-254 der Firma Merck verwendet; Laufmittel ist Toluol, Dioxan im Verhältnis 19:1.

*Beispiel 1*

14,8 Teile 1,4-Diamino-anthrachinon-2-sulfonsäure à 86,1% werden bei 60°C zu einer Mischung von 18 Teilen Wasser und 72 Teilen Acetamid gegeben. Nacheinander werden dazu 0,8 Teile Tris-(dioxa-3,6-heptyl)-amin, 0,8 Teile Soda, 4,0 Teile m-Nitrobenzolsulfonsäure und 15 Teile Natriumcyanid gegeben. Das Reaktionsgemisch wird bei 80°C während 4 Stunden gerührt. Das Produkt wird noch warm abgesaugt, mit warmem Wasser und mit wenig Methanol gewaschen. Nach dem Trocknen bei 60°C im Vakuum erhält man 12,0 Teile Rohprodukt, das laut Analyse 89,3% 1,4-Diamino-2,3-dicyanoanthrachinon enthält. Dies entspricht einer Reinausbeute von 92,7% d.Th., $R_f = 0,54$.

*Beispiel 2*

Wird Beispiel 1 in einer Lösungsmittel-Mischung bestehend aus 45 Teilen Acetamid und 45 Teilen Wasser wiederholt, so werden 12,37 Teile Produkt isoliert. Das rohe 1,4-Diamino-2,3-dicyanoanthrachinon hat laut Analyse einen Gehalt von 87,5%, was einer Reinausbeute von 93,7% d.Th. entspricht, $R_f$ = 0,54.

*Beispiel 3*

Wird Beispiel 1 in einer Lösungsmittel-Mischung bestehend aus 75 Teilen Formamid und 75 Teilen Wasser wiederholt, so werden 13,35 Teile Produkt isoliert. Das rohe Produkt hat laut Analyse einen Gehalt von 76,2%, was einer Reinausbeute von 88,1% d.Th. entspricht, $R_f$ = 0,54. Im Filtrat verbleiben etwa 5% d.TH. an Produkt.

*Beispiel 4*

22,8 Teile 1,4-Diamino-anthrachinon-2-sulfonsäure mit einem Gehalt von 78,1% werden bei Raumtemperatur in ein Gemisch aus 53 Teilen aus 53 Teilen 2-Pyrrolidon und 52 Teilen Wasser eingetragen. Der pH-Wert der Lösung wird mit 5 Teilen 24%iger wässriger Ammoniaklösung auf 7,5 bis 8 eingestellt. Anschliessend gibt man nacheinander 2 Teile Ammonium-hydrogencarbonat, 6,4 Teile 3-Nitrobenzol-1-sulfonsäure Natriumsalz und 9 Teile Natriumcyanid hinzu. Das Reaktionsgemisch wird anschliesend unter Rühren während 4 Stunden auf 88°C erhitzt und danach das Produkt noch warm abgesaugt, mit warmem 25%igem wässrigem 2-Pyrrolidon und schliesslich mit heissem Wasser gewaschen. Nach dem Trocknen des Produkts bei 50°C im Vakuum erhält man 14,9 Teile rohes 1,4-Diamino-2,3-dicyanoanthrachinon mit einem Gehalt von 95,5%; das entspricht einer Reinausbeute von 88,3% d.Th., $R_f$ = 0,54.

*Beispiel 5*

Wird Beispiel 4 in einer Lösungsmittel-Mischung bestehend aus 60 Teilen 2-Piperidon und 40 Teilen Wasser wiederholt, so werden 15,2 Teile Produkt isoliert. Das rohe 1,4-Diamino-2,3-dicyanoanthrachinon hat laut Analyse einen Gehalt von 93,0%, was einer Reinausbeute von 87,7% d.Th. entspricht, $R_f$ = 0,54.

*Beispiel 6*

65,2 Teile 1,4-Diamino-anthrachinon-2-sulfonsäure (Gehalt 78,1%) werden bei Raumtemperatur zu einer Mischung von 180 Teilen N-Methyl-acetamid und 180 Teilen Wasser gegeben. Nach dem Einstellen des pH-Wertes auf 7,5 bis 8 mit 15 Teilen wässriger Ammoniak-Lösung 24%ig werden nacheinander 18,2 Teile 3-Nitrobenzol-1-sulfonsäure · Natriumsalz und 25,5 Teile Natriumcyanid zugegeben. Das Reaktionsgemisch wird 4 Stunden bei 75°C gerührt. Das Produkt wird noch warm abgesaugt, und mit heissem Wasser gewaschen. Nach dem Trocknen bei 55°C im Vakuum erhält man 47,6 Teile Rohprodukt, das laut Analyse 91,1% 1,4-Diamino-2,3-dicyanoanthrachinon enthält. Dies entspricht einer Reinausbeute von 94,1% d.Th., $R_f$ = 0,54.

*Beispiel 7*

Wird Beispiel 6 in einer Lösungsmittel-Mischung bestehend aus 180 Teilen Propionsäureamid und 180 Teilen Wasser wiederholt, so werden 48,2 Teile Rohprodukt mit einem Gehalt von 90,5% isoliert, was einer Reinausbeute von 94,6% d.Th. entspricht, $R_f$ = 0,54.

**Patentansprüche**

1. Verfahren zur Herstellung von 1,4-Diamino-2,3-dicyanoanthrachinon durch Umsetzen von Diaminoanthrachinonen der Formel

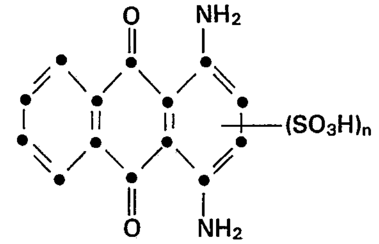

oder deren Salze, worin n 0, 1 oder 2 ist, mit einer Cyanidionen abgebenden Verbindung, in Gegenwart eines Oxidationsmittels, dadurch gekennzeichnet, dass man als Lösungsmittel ein Gemisch aus einem Carbonsäureamid, einem N-Alkylcarbonsäureamid oder einem Lactam und Wasser verwendet, das mehr als 10 Gewichtsprozent Wasser enthält, und in Abwesenheit von quaternären Ammoniumverbindungen arbeitet.

2. Verfahren gemäss Anspruch 1, dadurch gekennzeichnet, dass man als wasserhaltige, gegebenenfalls N-alkylierte Carbonsäureamide solche von Monocarbonsäuren einer Kettenlänge von 1 bis 6 verwendet.

3. Verfahren gemäss Anspruch 2, dadurch gekennzeichnet, dass man als Lösungsmittel ein Acetamid/Wasser-Gemisch verwendet.

4. Verfahren gemäss Anspruch 1, dadurch gekennzeichnet, dass man als Lösungsmittel wasserhaltige Lactame mit einer Ringgrösse von 5 bis 7 verwendet.

5. Verfahren gemäss Anspruch 4, dadurch gekennzeichnet, dass man als Lösungsmittel ein Gemisch aus 2-Pyrrolidon und Wasser verwendet.

6. Verfahren gemäss den Ansprüchen 1 bis 5, dadurch gekennzeichnet, dass man als Lösungsmittel ein Carbonsäureamid bzw. Lactam/Wasser-Gemisch mit einem Wassergehalt von 10 bis 60 Gewichtsprozent verwendet.

7. Verfahren gemäss Anspruch 1, dadurch gekennzeichnet, dass man als Cyanidionen abgebendes Mittel Natrium- oder Kaliumcyanid verwendet.

8. Verfahren gemäss Anspruch 1, dadurch gekennzeichnet, dass man als Oxidationsmittel Nitrobenzol oder m-Nitrobenzolsulfonsäure verwendet.

9. Verfahren gemäss Anspruch 1, dadurch gekennzeichnet, dass man dem Reaktionsgemisch ein säurebindendes Mittel, insbesondere Soda, Natriumhydrogencarbonat, wässriges Ammoniak und/oder ein Mono-, Di- oder Trialkylamin zusetzt.

10. Verfahren gemäss den Ansprüchen 1 bis 9, dadurch gekennzeichnet, dass man als Ausgangsmaterial 1,4-Diaminoanthrachinon-2-sulfonsäure verwendet.

**Claims**

1. A process for the preparation of 1,4-diamino-2,3-dicyanoanthraquinone by reacting a diaminoanthraquinone, or a salt thereof, of the formula

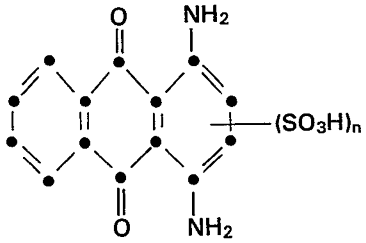

wherein n is 0, 1 or 2, with a compound which donates cyanide ions, in the presence of an oxidising agent, which comprises using as solvent a mixture of a carboxamide, an N-alkylated carboxamide or a lactam and water, which mixture contains more than 10% by weight of water, the process being carried out in the absence of quaternary ammonium compounds.

2. A process according to Claim 2, which comprises using as water-containing, optionally N-alkylated carboxamide an amide of a monocarboxylic acid containing 1 to 6 atoms in the chain.

3. A process according to Claim 2, which comprises using a mixture of acetamide/water as solvent.

4. A process according to Claim 1, which comprises using a water-containing lactam containing 5 to 7 members in the ring as solvent.

5. A process according to Claim 4, with comprises using a mixture of 2-pyrrolidone and water as solvent.

6. A process according to any one of Claims 1 to 5, which comprises using as solvent a mixture of carboxamide or lactam and water, said mixture having a water content of 10 to 60% by weight.

7. A process acording to Claim 1, which comprises using sodium cyanide or potassium cyanide as agent which donates cyanide ions.

8. A process according to Claim 1, which comprises using nitrobenzene or m-nitrobenzenesulfonic acid as oxidising agent.

9. A process according to Claim 1, wherein an acid acceptor, in particular sodium carbonate, sodium bicarbonate, aqueous ammonia and/or a mono-, di- or trialkylamine is added to the reaction mixture.

10. A process according to any one of Claims 1 to 9, wherein 1,4-diaminoanthraquinone-2-sulfonic acid is used as starting material.

**Revendications**

1. Procédé pour la préparation de la 1,4-diamino-2,3-dicyanoanthraquinone par réaction de diamino-anthraquinones de formule

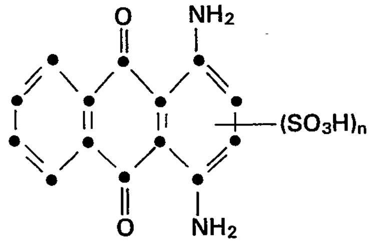

dans laquelle n vaut 0, 1 ou 2, ou de leurs sels, avec un composé donneur d'ions cyanure, en présence d'un oxydant, caractérisé par le fait que l'on utilise en tant que solvant un mélange d'un amide d'acide carboxylique, d'un N-alkyl-amide d'acide carboxylique ou d'un lactame, et d'eau qui contient plus de 10% en poids d'eau, et on opère en absence de composés d'ammonium quaternaire.

2. Procédé selon la revendication 1, caractérisé par le fait que l'on utilise en tant qu'amides d'acides carboxyliques éventuellement N-alkylés, hydratés, ceux d'acides monocarboxyliques à longueur de chaîne allant de 1 à 6.

3. Procédé selon la revendication 2, caractérisé par le fait que l'on utilise en tant que solvant un mélange d'acétamide et d'eau.

4. Procédé selon la revendication 1, caractérisé par le fait que l'on utilise en tant que solvant des lactames hydratés ayant un cycle à 5-7 chaînons.

5. Procédé selon la revendication 4, caractérisé par le fait que l'on utilise en tant que solvant un mélange de 2-pyrrolidone et d'eau.

6. Procédé selon les revendications 1 à 5, caractérisé par le fait que l'on utilise en tant que solvant un mélange d'amide d'acide carboxylique ou de lactame et d'eau, ayant une teneur en eau de 10 à 60% en poids.

7. Procédé selon la revendication 1, caractérisé par le fait que l'on utilise, en tant qu'agent donneur d'ions cyanure, le cyanure de sodium ou le cyanure de potassium.

8. Procédé selon la revendication 1, caractérisé par le fait que l'on utilise en tant qu'oxydant le nitrobenzène ou l'acide m-nitrobenzènesulfonique.

9. Procédé selon la revendication 1, caractérisé par le fait que l'on ajoute au mélange réactionnel un agent capteur d'acide, en particulier du carbonate de sodium, de l'hydrogénocarbonate de sodium, de l'ammoniac en solution aqueuse et/ou une mono-, di- ou trialkylamine.

10. Procédé selon les revendications 1 à 9, caractérisé par le fait que l'on utilise en tant que produit de départ l'acide 1,4-diaminoanthraquinone-2-sulfonique.